Europäisches Patentamt

European Patent Office

Office européen des brevets

(11) Veröffentlichungsnummer: **0 340 324 B1**

# EUROPÄISCHE PATENTSCHRIFT

(45) Veröffentlichungstag der Patentschrift: **16.12.92**

(51) Int. Cl.⁵: **C07C 43/04**, C07C 41/09, C07C 41/42

(21) Anmeldenummer: **88107118.7**

(22) Anmeldetag: **04.05.88**

(54) **Verbessertes Verfahren zur Herstellung von reinem Dimethylether.**

(43) Veröffentlichungstag der Anmeldung:
**08.11.89 Patentblatt 89/45**

(45) Bekanntmachung des Hinweises auf die
Patenterteilung:
**16.12.92 Patentblatt 92/51**

(84) Benannte Vertragsstaaten:
**AT BE CH DE ES FR GB GR IT LI LU NL SE**

(56) Entgegenhaltungen:
**EP-A- 0 099 676**
**EP-A- 0 124 078**
**DE-B- 1 050 750**
**US-A- 3 296 314**
**US-A- 4 665 249**

(73) Patentinhaber: **RWE-DEA Aktiengesellschaft
für Mineraloel und Chemie
Überseering 40
W-2000 Hamburg 60(DE)**

(72) Erfinder: **Meisenburg, Ewald
Höhenring 60
W-5357 Swisttal-Heimerzheim(DE)**
Erfinder: **Dornhagen, Horst, Dr.
Auf dem Radacker 21
W-5047 Wesseling(DE)**
Erfinder: **Hammer, Hartmut, Dr.
Leyboldstrasse 21
W-5000 Köln 1(DE)**

(74) Vertreter: **Schupfner, Gerhard D.
Müller, Schupfner & Gauger Karlstrasse 5
Postfach 17 53
W-2110 Buchholz/Nordheide(DE)**

## Beschreibung

Die Erfindung betrifft ein verbessertes Verfahren zur Herstellung von reinem Dimethylether durch katalytische Dehydratisierung von Methanol über einem $\gamma$-$Al_2O_3$-Katalysator, der bevorzugt geringe Mengen $SiO_2$ enthält, und Reinigung des Dehydratisierungsprodukts durch Zuführung desselben in eine Destillationskolonne zur Gewinnung von reinem Dimethylether an bestimmten Böden dieser Kolonne und Entnahme des reinen Dimethylethers und von Verunreinigungen oberhalb bestimmter Böden der gleichen Kolonne, wobei zusätzlich zum Dehydratisierungsprodukt eine Waschflüssigkeit eingesetzt wird.

Bis zur Entwicklung der Methanol-Niederdruck-Synthese-Verfahren wurde Dimethylether als Nebenprodukt der Methanol-Hochdruck-Synthese in einer Menge von etwa 2 - 5 Gew.-% erhalten, bezogen auf die Gesamtmenge des Synthesereaktor-Ausgangsprodukts und bei der Gewinnung von Reinmethanol destillativ aus dem weitere Verunreinigungen enthaltenden Vorlauf abgetrennt.

Nach Einführung der Niederdruck-Methanolsynthese-Verfahren, bei denen keine nennenswerten Mengen an Dimethylether anfallen, wurden Syntheseverfahren zur Herstellung von Dimethylether durch katalytische Dehydratisierung von Methanol entwickelt. Zahlreiche Verfahren sind in der Patentliteratur beschrieben worden. So lassen sich nach DE-PS 680 328 aliphatische Ether durch Erhitzen von Alkoholen in Gegenwart von Zinkchlorid erzeugen.

Weitere geeignete Katalysatoren zur Herstellung von Ethern aus Alkoholen sind gemäß GB-PS 332 756, GB-PS 350 010, GB-PS 403 402, US-PS 1 873 537 und FR-PS 701 335 Eisenchlorid, Kupfersulfat, Zinnchlorid, Manganchlorid, Aluminiumchlorid und -sulfat, Chromsulfat, Alaune, Thorium-Verbindungen, Aluminiumoxid, Titanoxid, Bariumoxid, Silicagel oder Aluminiumphosphat. In "Industrial and Engineering Chemistry", Vol. 41, No. 12, S. 2928, 1949 wird die Verwendung von Bauxiten mit $SiO_2$-Gehalten von 4,40 - 13,99 Gew.-% beschrieben. In US-PS 3 036 134 wird ein Aluminiumsilikat-Katalysator offenbart, zur Herstellung von Dimethylether aus Methanol, der ein $Al_2O_3$:$SiO_2$-Verhältnis von 1 Teil zu 1,35 bis 0,3 Teilen aufweist. Auch die Synthese von Dimethylether direkt aus Synthesegas (CO + $H_2$) ist beschrieben worden (DE-PS 23 62 944, DE-PS 27 57 788 und DE-PS 32 20 547).

Als technisch wichtigste Katalysatoren haben sich gemäß DE-PS 28 18 831, DE-OS 32 01 155, EP-A 0 099 676 und EP-A 0 124 078 insbesondere Aluminiumoxid- und Aluminiumsilikat-Katalysatoren mit und ohne Dotierung durchgesetzt. In DE-PS 28 18 831 wird ein Katalysator zur Herstellung von Dimethylether offenbart, der jedes beliebige Aluminiumoxid-Grundmaterial mit genügend großer Oberfläche enthalten kann, in dem grundsätzlich seltene Erden mit 1 - 30 Gew.% $SE_2O_3$ vorhanden sind. Schließlich ist in EP-A 0 099 676 ein Katalysator beschrieben, der 1 - 20 Gew.-% $SiO_2$, vorzugsweise 1 - 10 Gew.-% $SiO_2$ und noch bevorzugter 6 Gew.-% $SiO_2$ enthält. Der hierbei anfallende rohe Dimethylether enthält das Reaktionswasser, nicht umgesetztes Methanol sowie kleine Mengen an Verunreinigungen, wie beispielsweise Methylformiat, Kohlenwasserstoffe, Amine und Sulfide, Carbonsäuren, Ester, Amide, Acetale u.a.

In diesen Synthese-Anlagen wird der rohe Dimethylether in zwei hintereinander geschalteten Destillationskolonnen aufgearbeitet, wobei in der ersten, unter Druck betriebenen Kolonne Dimethylether gewonnen wird und in der zweiten Kolonne nicht umgesetztes Methanol zurückgewonnen wird.

So wird in EP-A 0 124 078 ein Verfahren beschrieben, wonach in einer ersten unter Druck stehenden Kolonne, Dimethylether im Seitenabzug entnommen wird, während in einer zweiten unter niedrigerem Druck arbeitenden Kolonne über Kopf die zwischen Dimethylether und Methanol siedenden Verunreinigungen abgenommen werden. Methanol wird ebenfalls aus der 2. Kolonne im Seitenabzug entnommen. Als Katalysator können $Al_2O_3$, $SiO_2$, Aluminiumsilikat und bevorzugt $\gamma$-Aluminiumoxid eingesetzt werden.

Obgleich bei diesem Verfahren ein gegenüber dem Stand der Technik reinerer Dimethylether gewonnen werden kann, hat es den erheblichen wirtschaftlichen Nachteil, daß nicht nur die erste, sondern auch die 2. Destillationskolonne mit hoher Bodenzahl ausgestattet sein muß, so daß sowohl hohe Investitionskosten als auch insbesondere hohe Betriebskosten enstehen und daß ferner die Gefahr besteht, daß die zwishen Dimethylether und Methanol siedenden Verunreinigungen nicht vollständig in die 2. Kolonne gelangen, sondern nach Anreicherung in der 1. Kolonne mit dem Dimethylether abgezogen werden, so daß keine Geruchsfreiheit vorliegt.

In der europäischen Patentanmeldung Nr. 87116477.8 wird ein Verfahren beschrieben, gemäß dem Dimethylether aus Methanol in Gegenwart eines $\gamma$-$Al_2O_3$-Katalysators mit sehr geringem $SiO_2$-Gehalt erzeugt wird und in einem destillativen Reinigungsverfahren ein hochreiner, geruchsfreier Dimethylether gewonnen wird, wobei die zwischen Dimethylether und Methanol siedenden Verunreinigungen in der gleichen Kolonne an bestimmten Böden im Seitenabzug abgetrennt werden.

In der US-PS 3 296 314 ist ein Verfahren beschrieben, bei dem Stickoxide enthaltender Dimethylether in der Gasphase mit einer wässrigen Säurelösung, die zusätzlich eine $NH_2$-Verbindung enthält, gewaschen wird.

Gemäß US-PS 4 665 249 wird ein im wesentlichen wasserfreier, Dimethylether enthaltender Methanol-Produktstrom gewonnen. Hierbei wird Rohmethanol dem Einsatzprodukt, das Dimethylether, Wasser und Methanol enthält, über einen höher gelegenen Boden der Destillationskolonne zugeführt.

Da Dimethylether als Treibmittel für Aerosolsprays zunehmend an Bedeutung gewinnt, werden für bestimmte Anwendungen sehr hohe Reinheitsanforderungen an dieses Produkt gestellt.
So dürfen insbesondere für die kosmetische, human- und Haus-halts-Anwendung keine irritierenden Substanzen im Dimethyl-ether enthalten sein. Ferner muß Dimethylether hierbei frei bzw. weitgehend frei von Geruchsstoffen sein.

Es bestand daher die Aufgabe, die Gewinnung von hochreinem Dimethylether weiter zu verbessern.
Die Lösung dieser Aufgabe ist der Anmelderin in hervorragender Weise durch das erfindungsgemäße Verfahren gelungen durch die Herstellung von reinem Dimethylether durch katalytische Dehydratisierung von Methanol bei einer Temperatur von 14o° - 5oo°C und einem Druck von 1 bis 5o bar und destillative Aufarbeitung des Dehydratisierungsprodukts dadurch gekennzeichnet, daß

a, das Dehydratisierungsprodukt in die Kolonne zur Gewinnung von reinem Dimethylether unterhalb des 25. Bodens (vom Kopf der Kolonne) an einem oder mehreren Böden in die Kolonne und mindestens 1 Boden oberhalb des Kolonnensumpfes zugeführt wird,

b, der reine Dimethylether ab dem 15. Boden, bevorzugt 10. Boden (vom Kopf der Kolonne) an einem Boden oder mehreren Böden abgezogen wird,

c, daß ein Verunreinigungen, die höher als Dimethylether und niedriger als Methanol sieden, enthaltenes Destillat an einem Boden oder mehreren Böden abgezogen wird, der (die) um mindestens 3 Böden oberhalb des (obersten) Zulaufs des den Dimethylether enthaltenden Einsatzgemisches und um mindestens einen Boden unterhalb des 15. Bodens (vom Kopf der Kolonne) liegt (liegen), wobei der Abstand zwischen dem Abzugsboden für reinen Dimethylether und Verunreinigungen bevorzugt wenigstens 5 Böden beträgt,

d, zusätzlich zum Dehydratisierungsprodukt aus dem Dimethylether-Synthesereaktor eine Waschflüssigkeit eingesetzt wird,

wobei bei einem Gesamt-Einsatzprodukt, einschließlich Waschflüssigkeitszusats gemäß d, das >0 - 5 Gew.-% Dimethylether enthält, ein Rückflußverhältnis von 1:0,2 bis 1:100, bevorzugt 1:1 bis 1:25 gefahren wird, bei einem Gesamt-Einsatzprodukt, das 20 - 80 Gew.-% Dimethylether enthält, ein Rückflußverhältnis von 1:0,2 bis 1:50 gefahren wird, bevorzugt von 1:0,5 bis 1:10 und besonders bevorzugt von 1:1 bis 1:5, bei einem Gesamt-Einsatzprodukt, das >5 - <20 Gew.-% Dimethylether enthält, ein Rückflußverhältnis von 1:0,2 bis 1:50 und bevorzugt von 1:0,4 bis 1:25 und bei einem Gesamt-Einsatzprodukt, das >80 Gew.-% bis < 99 Gew.-% Dimethylether enthält, ein Rückflußverhältnis von 1:0,2 bis 1:50, bevorzugt von 1:0,2 bis 1:10 und besonders bevorzugt von 1:0,4 bis 1:5 gefahren wird bzw. bei > 80 Gew.-% bis 99 Gew.-% Dimethylether 1:>5 bis 1:50.
Ein sehr reiner Dimethylether kann auch erfindungsgemäß hergestellt werden, durch katalytische Dehydratisierung von Methanol bei einer Temperatur von 14o - 5oo°C und einem Druck von 1 bis 5o bar und destillative Aufarbeitung des Dehydratisierungsprodukts, wobei das Dehydratisierungsprodukt in die Kolonne zur Gewinnung von reinem Dimethylether unterhalb des 25. Bodens (vom Kopf der Kolonne) an einem oder mehreren Böden in die Kolonne eingesetzt wird und das Dehydratisierungsprodukt mindestens 1 Boden oberhalb des Kolonnensumpfes zugeführt wird, der reine Dimethylether ab dem 15. Boden, bevorzugt ab dem 1o. Boden (vom Kopf der Kolonne) an einem Boden oder mehreren Böden abgezogen wird, ein Verunreinigungen, die höher als Dimethylether und niedriger als Methanol sieden, enthaltendes Destillat an einem Boden oder mehreren Böden abgezogen wird, der (die) um mindestens 3 Böden oberhalb des (obersten) Zulaufs des den Dimethylether enthaltenden Einsatzgemisches und um mindestens einen Boden unterhalb des 15. Bodens (vom Kopf der Kolonne) liegt (liegen), wobei bei einem Einsatzprodukt, das >o - 5 Gew.-% Dimethylether enthält, ein Rückflußverhältnis von 1:o,2 bis 1:<1 gefahren wird,

bei einem Einsatzprodukt, das 2o-8o Gew.-% Dimethylether enthält, ein Rückflußverhältnis von 1:o,2 bis 1: <o,4 oder von 1: >5 bis 1:5o gefahren wird und bei einem Einsatzprodukt, das >5 - <2o Gew.-% Dimethylether enthält ein Rückflußverhältnis 1:o,2 bis 1: <o,4 oder 1: >25 bis 1:5o gefahren wird.

Die Bestimmung geruchsbelästigender Substanzen erfolgt noch immer überwiegend empirisch, wobei direkte sensorische Messungen durch ein geübtes Team erfolgen. So wurde beispielsweise die Belästigungsschwelle "BS" für $H_2S$ durch ein Testkollektiv von 15o Personen auf 45 $\mu g/m^3$ ermittelt (Schriftenreihe der Landesanstalt für Immissionsschutz des Landes Nordrhein-Westfalen, Heft 49 (1979) S.77).

In den Fällen, in denen die Geruchsschwelle im durch Instrumente messbaren Bereich liegt, können Geruchsschwellen bzw. Belästigungsschwellen auch durch Messmethoden wie Gaschromatographie, elektrische Leitfähigkeit, Photometrie oder Fluoreszenzmessung bestimmt werden ("Erdöl und Kohle-Erdgas-Petrochemie", Bd. 32, Heft 2, Febr. 1975, S.86).

Die Geruchsbestimmungen im Falle der vorliegenden Erfindung beruhen auf der sensorischen Methode.

Sowohl Rohmethanol und Reinmethanol aus einer Methanolsyntheseanlage als auch katalytisch aus Methanol hergestellter Dimethylether enthalten wie bereits oben ausgeführt, zahlreiche Verunreinigungen, die teilweise sehr geruchsintensiv sind. Während in Methanol-Hochdrucksyntheseanlagen ein Rohmethanol anfällt, in dem im allgemeinen 2-3, jedoch bis zu 5 Gew.-% Dimethylether enthalten sein können, werden bei Dimethylethersynthesen oder Roh- oder Reinmethanol je nach Fahrweise im allgemeinen 20 bis 80 Gew.-% Dimethylether aus dem eingesetzten Methanol am Reaktorausgang erhalten, Zusätzlich sind im rohen Dimethylether die oben genannten Verunreinigungen, Reaktionswasser und nicht umgesetztes Methanol enthalten.

Da die Seidepunkte der Verunreinigungen, wie beispielsweise von Dimethylamin (Kp 6,9 °C), Dimethylsulfid (Kp 37,3 °C), Methylmercaptan (5,8 °C), Ameisensäure (Kp 100,75 °C), Ameisensäuremethylester (Kp 31,5 °C), Formaldehyd (-21 °C), Formaldehyddimethylacetal (Kp 45,5 °C), oder Essigsäuremethylester (Kp 56,95 °C) sowie ihre Löslichkeiten und Dampfdrucke im Produktgemisch sehr unterschiedlich sind und die subjektiv wahrgenommene Geruchsintensität der einzelnen Verunreinigungen ebenfalls sehr unterschiedlich ist und zusätzlich eine Reihe azeotroper Gemische gebildet werden, wird die Lösung der Aufgabe, hochreinen Dimethylether in hohen Ausbeuten nach einem gegenüber dem Stand der Technik wirtschaftlicheren Verfahren zu erzeugen, sehr erschwert.

Die Anmelderin hat daher in mehrjährigen Untersuchungen durch zahlreiche Testreihen in Labor, Technikum und technisher Anlage überraschend gefunden, daß hochreiner Dimethylether praktisch quantitativ in einer besonders wirtschaftlichen Weise durch die erfindungsgemäßen Katalysatoren und die erfindungsgemäße destillative Reinigung erhalten werden kann.

In Figur 1 ist beispielhaft eine Anlage zur Erzeugung und Reinigung von Dimethylether dargestellt.

In Figur 2 ist beispielhaft eine Destillationskolonne für die Gewinnung von reinem Dimethylether dargestellt.

In Figur 3 ist beispielhaft diese Kolonne mit einem Vorlauf-Seitenstripper dargestellt.

Die in den Ansprüchen 2, 3, und 4 offenbarten erfindungsgemäßen Katalysatoren sind Katalysatoren vom $\gamma$-$Al_2O_3$-Typ. Sie enthalten im Unterschied zu den Katalysatoren des Standes der Technik nur sehr geringe Mengen $SiO_2$ und führen bezüglich Geruchsfreiheit zu wesentlich besseren Ergebnissen als die bekannten Katalysatoren. Der $SiO_2$-Gehalt ist >0 bis 1 Gew.-%. Bevorzugt ist eine Menge an $SiO_2$ von 0,001 bis 0,5 Gew.-% und besonders bevorzugt von 0,001 bis 0,2 Gew.-% $SiO_2$.

Die erfindungsgemäßen Katalysatoren können zusätzlich weitere Komponenten in kleinen und Kleinst-Mengen enthalten, wie z.B. $Na_2O$ oder andere Alkali- und Erdalkalioxide, Alkali-, Erdalkali- oder Aluminiumsulfate, Eisenoxid, Kobaltoxid, Nikkeloxid, Titanoxid, seltene Erden und andere Verbindungen.

Die Umsetzung an den erfindungsgemäßen Katalysatoren erfolgt bei einer Temperatur von 140 bis 500 °C, bevorzugt von 150 viw 450°C und einem Druck von 1 bis 50 bar, bevorzugt von 3 bis 25 bar. Die Umsetzung kann in der Gas- oder Flüssigphase erfolgen, bevorzugt ist die Umsetzung in der Gasphase. Es ist von Vorteil, die Drükke im Synthesereaktor und der destillativen Aufarbeitung des Dimethylethers aneinander anzupassen. Es wird bei Katalysatorbelastungen von 0,2 - 16 l/l h, bevorzugt von 0,5 - 13,5 l/l h gearbeitet.

Jedoch auch in Gegenwart sonstiger Katalysatoren, z.B. von $\gamma$-$Al_2O_3$ mit höheren $SiO_2$-Gehalten, kann gemäß Anspruch 1 der vorliegenden Erfindung die Qualität des Dimethylethers wesentlich vervessert werden.

Obgleich mit dem erfindungsgemäßen Verfahren diskontinuierlich gearbeitet werden kann, ist die kontinuierliche Fahrweise bevorzugt.

Als Reaktoren für die erfindungsgemäße Umsetzung können beispielsweise die dem Stand der Technik entsprechenden Festbett-, Fließbett- und Wirbelbett-Reaktoren eingesetzt werde. Auch abgewandelte Reaktoren, die für Umsetzungen in Gegenwart von Katalysatoren geeignet sind, können erfindungsgemäß eingesetzt werden.

Die Umsetzung in Gegenwart der erfindungsgemäßen Katalysatoren kann im geraden Durchgang kinetisch oder thermodynamisch bestimmt sein in Abhängigkeit von den Reaktionsparametern, hierbei können jeweils die enstpechenden Mengen Dimethylether neben nicht umgesetztem Methanol am Reaktorausgang erhalten werden.

Die Untersuchungen der Anmelderin haben zu dem Ergebnis geführt, daß zur Lösung der Aufgabe, auf besonders wirtschaftliche Weise hochreinen geruchsfreien Dimethylether mit nahezu quantitativer Ausbeute gewinnen zu können, die Zuführung des Dehydratisierungsproduktes in die Destillationskolonne zur Gewinnung reinen Dimethylethers unterhalb des 25. Bodens (vom Kopf der Kolonne) an einem oder mehreren Böden erfolgen muß. Wird das Einsatzprodukt an einem höheren Boden zugeführt, so erhält man nicht die gewünschte Reinheit, insbesondere nicht die gewünschte Geruchsfreiheit, sofern man nicht an den zum Abzug der Verunreinigungen bestimmten Böden relativ große Mengen an Dimethylether mitabzieht. Das Einsatzprodukt kann z.B. erfindungsgemäß auch an mehreren Böden je nach Bodenzahl der Kolonne zwischen dem 25. und 5o. Boden (vom Kopf der Kolonne) zugeführt werden. So kann beispielsweise bei einer Kolonne, die insgesamt 6o Böden enthält, zwischen dem 32. und 46. Boden das Einsatzprodukt zugeführt werden.

Da die Zuführung mindestens 1 Boden, bevorzugt 5 Böden und besonders bevorzugt 10 Böden oberhalb des Kolonnensumpfes liegen muß, kann (können) die Zuführung(en) im Bodenbereich unterhalb des 25. Bodens (vom Kopf der Kolonne) und oberhalb des 1., bevorzugt 5. und besonders bevorzugt 10. Bodens (vom Sumpf der Kolonne) gewählt werden. Ferner muß erfindungsgemäß der reine Dimethylether ab dem 15. Boden, bevorzugt ab dem 10. Boden (vom Kopf der Kolonne) an einem oder mehreren Böden abgezogen werden. Dies kann beispielsweise der 6. Boden sein, es kann jedoch auch der 1. bis 5. Boden sein bzw. ein Boden zwischen dem 6. und 15. Boden sein, es kann jedoch ggfs. auch das Kopfkondensat z.B. bei Einsatz einer Vorlaufkolonne abgezogen werden. Es Können auch mehrere Böden in diesen Bereichen sein.

Die Untersuchungen der Anmelderin haben ferner ergeben, daß Komponenten mit Siedepunkten zwischen denen von Methanol und Dimethylether als Destillat (gasförmig oder flüssig) in der gleichen Kolonne an einem Boden oder mehreren Böden abgezogen werden, der bzw. die um mindestens 3 Böden oberhalb des (obersten) Zulaufs für das den verunreinigten Dimethylether enthaltenden Dehydratisierungsprodukts,das Dimethylether, Methanol und Verunreinigungen enthält, jedoch unterhalb des 15. Bodens (vom Kopf der Kolonne) liegt bzw. liegen.

Es ist erfindungsgemäß möglich, Verunreinigungen in diesem Bereich der Kolonne auch an mehreren Böden gasförmig und/oder flüssig abzuziehen, wobei naturgemäß um so mehr Dimethylether in den abgezogenen Verunreinigungen enthalten ist, je näher der Boden für den Abzug der Verunreinigungen am Boden bzw. an den Böden für den Abzug des reinen Dimethylethers liegt. Es wird daher bevorzugt die Hauptmenge der Verunreinigungen in einem Abstand von etwa mindestens 5 Böden vom Dimethyletherabzug entnommen. Es kann gleichzeitig auch weiteres verunreinigtes Produkt im Bedarfsfall an weiteren Abnahmestellen mit grösserem Abstand als 5 Böden entnommen werden. Zusätzlich kann auch ein Teil an Verunreinigungen unterhalb der Einspritzung abgezogen werden.

Erfindunsgemäß wird zusätzlich zum in dem Dehydratisierungsprodukt enthaltenen Methanol, eine Waschflüssigkeit eingesetzt, die bevorzugt Methanol ist.

Es kann sich hierbei um reines Methanol, um sog.Rohmethanol oder auch um andere Methanolsorten handeln.

Unter Rohmethanol wird Methanol verstanden, das direkt aus einer Methanolsynthese stammt. Hierbei kann es sich um eine Niederdruck-, Mitteldruck- oder Hochdruck-Methanolsynthese handeln, während reines Methanol durch Destillation gereinigtes Methanol ist, das den nationalen und internationalen Spezifikationen entspricht. Eine andere Methanolsorte kann beispielsweise ein in einer Vorlaufkolonne abgetopptes Rohmethanol sein, aus dem Gase wie $CH_4$, $CO_2$, $N_2$ u.a. entfernt worden sind.

Die Waschflüssigkeit kann jedoch auch Wasser oder Methanol/Wasser-Gemisch oder auch Dimethylether im Gemisch mit Wasser und/oder Methanol sein.

Die Waschflüssigkeit kann, bevor sie in die Dimethylether-Destillationskolonne gelangt, dem Dehydratisierungsprodukt zugemischt werden, so daß das Gemisch auf den bzw. die Einspritzböden gelangt.

Grundsätzlich kann auch das Einsatzprodukt zumindest teilweise gasförmig in die Kolonne eingesetzt werden.

Es kann auch eine Fahrweise gewählt werden, bei der die Waschflüssigkeit getrennt bzw. zumindest teilweise getrennt auf den gleichen Einspritzboden bzw. die gleichen Einspritzböden geleitet werden, auf den bzw. die auch das Dehydratisierungsprodukt geleitet wird.

Eine weitere erfindungsgemäße Fahrweise ist, daß die Waschflüssigkeit an anderen Böden als das Dehydratisierungsprodukt zugeführt wird. Hierbei kann es sich um allen Böden einschließlich dem 3. Boden unterhalb des untersten Dimethyletherabzugs bis zu dem 3. Boden vom Sumpf der Kolonne aus handeln. Bevorzugt wird die Waschflüssigkeit unterhalb des untersten Zuführungsbodens für das Dehydratisierungsprodukt eingesetzt.

Erfindungsgemäß ist es auch möglich das heiße, aus dem Dimethylether-Synthesereaktor austretende Produkt in einer vor der Destillationskolonne befindlichen Waschvorrichtung zumindest teilweise mit Waschflüssigkeit zu waschen. Während das gewaschene gasförmige Dehydratisierungsprodukt zur Destillationskolonne strömt, wird die Verunreinigungen enthaltende Waschflüssigkeit abgezogen und kann dem unteren Teil der Destillationskolonne zugeführt werden.

Der Waschflüssigkeitszusatz hat die vorteilhafte Wirkung eines zusätzlichen Reinigungseffektes bezüglich des zu gewinnenden hochreinen Dimethylethers. Die Menge der zugesetzten Waschflüssigkeit kann daher in sehr weiten Grenzen von >0%, bezogen auf die Menge des eingesetzten Dehydratisierungsproduktes, bis zu einem mehrfachen dieser Menge variieren. Maßgebend ist in erster Linie die Qualität des gowonnenen Dimethylethers. Sollten Verunreinigungen bzw. störender Geruch in diesem auftreten, so ist die Menge der zugesetzten Waschflüssigkeit zu erhöhen. Ein weiteres Mengenkriterium ist die Produktionsmenge an Rohmethanol im Falle der Integration der Dimethyletherdestillation in eine Rohmethanoldestillation.

Je nach Produktionskapazität der Methanolsyntheseanlage und der Dimethylethersyntheseanlage kann bei gemeinsamer Destillation des Rohmethanols und Dehydratisierungsprodukts aus der Dimethylethersyntheseanlage in einer Kolonne gemäß vorliegender Erfindung, in der an den genannten Böden der reine Dimethylether abgezogen wird, das Mengenverhältnis Rohmethanol zu Dehydratisierungsrprodukt sehr stark variieren.
Dies wird für den Fachmann deutlich, wenn man davon ausgeht, daß Methanolsyntheseanlagen meist eine Jahreskapazität von 100.000 t bis 600.ooo t besitzen, während Jahresproduktions-Kapazitäten von Dimethylethersyntheseanlagen etwa zwischen 5.000 t bis 100.000 t liegen. Beispielsweise kann das Verhältnis bei einer Dimethyletheranlage einer Kapazität von 5.000 t/a und einer Methanolkapazität von 5000.000 t/a bei 1:100 liegen. Andererseits kann die Waschflüssigkeit nur ein Bruchteil der Menge an Dehydratisierungsprodukt sein, wie z.B. 1/100, 1/20, 1/10 oder 1/5. Gleiches gilt für den Einsatz der anderen Waschflüssigkeiten.
Es kann weiterhin von Vorteil sein, im Sumpf dieser Destillationskolonne, insbesondere bei Einsatz von Rohmethanol, einen bestimmten pH-Wert einzustellen durch Zugabe einer Base, insbesondere von NaOH, KOH, und anderen Alkali- und Erdalkalibasen. Die Zuführung kann grundsätzlich an einer beliebigen Stelle erfolgen.

Auf einfache Weise läßt sich die Base in die zur Kolonne führende Leitung für das Dehydratisierungsprodukt bzw. für Dehydratisierungsprodukt und Methanol oder in die Methanolzuführungsleitung zugeben. Hierbei tritt die gewünschte Durchmischung bereits in der Leitung auf. Die Zugabe einer Base ist insbesondere von Vorteil bei Vorliegen einer größeren Menge des leicht siedenden Methylformiats, das somit nicht oder nur teilweise in den oberen Teil der Dimethylether-Destillationskolonne gelangt.

Eine Base kann auch bereits in einer Vorlaufkolonne zugegeben werden. Hierdurch ist es möglich, Amin-Verunreinigungen bereits in der Vorlaufkolonne abzutrennen.

Setzt man ein Einsatzprodukt ein, das beispielsweise> 0 - 5 Gew.-% Dimethylether enthält, neben Methanol, sonstigen Komponenten mit Siedepunkten zwischen denen von Methanol und Dimethylether und ggfs. Wasser und sonstigen Sauerstoff enthaltenden Kohlenwasserstoffen oder Verbindungen, die im Rohmethanol enthalten sind, wie beispielsweise Alkoholen mit einer Anzahl C-Atomen>1, so wird die Kolonne mit einem Rückflußverhältnis von 1:0,2 bis 1:100, bevorzugt von 1:1 bis 1:25 betrieben in Abhängigkeit von dem Dimethyletheranteil. So kann beispielsweise bei einem Dimethylethergehalt von 1 Gew.-% das Rückflußverhältnis 1:20 betragen. Ein Rückflußverhältnis von beispielsweise 1:1 bedeutet, daß die Menge an abgezogenem Dimethylether = 1 Teil ist und die Dampfmenge zur Kondensation am Kopf der Kolonne = 1 + 1 Teile ist, (1. Zahl stellt die abgezogene Menge dar). Ein Rückflußverhältnis von 1:5-8 ist beispielsweise bevorzugt bei einem Dimethylethergehalt von 3-4 Gew.-%. Diese Dimethylethermenge entspricht den Gehalten, wie sie bei Hochdruck-Methanolsynthesen anfallen. Der Dimethylether kann jedoch nur dann in hochreiner, nahezu quatitativer Menge gewonnen werden, wenn man die erfindungsgemäß genannte Zuführung und die erfindungsgemäßen Abnahmestellen von Dimethylether und Verunreinigungen enthaltendem Destillat wählt. Enthält das Einsatzgemisch beispielsweise 20-80 Gew.-% Dimethylether sowie Methanol und sonstige Komponenten mit Siedepunkten zwischen denen von Methanol und Dimethylether und ggfs. Wasser und sonstige Sauerstoff enthaltende Kohlenwasserstoffe, wie beispielsweise Alkohole mit einer Anzahl C-Atomen > 1, so ist das Rückflußverhältnis auf 1:0,2 bis 1:50, bevorzugt von 1:0,5 bis 1:10 und besonders bevorzugt von 1:1 bis 1:5 einzustellen je nach Dimethylethergehalt. Beispielsweise kann man bei einem Gemisch, das 60 Gew.-% Dimethylether und 15 Gew.-% Methanol enthält, neben Wasser und Verunreinigungen, ein Rückflußverhältnis von 1:1,5 bis 1:2,5 einstellen.

Solche Einsatzgemische sind typische Gemische, wie sie bei der katlaytischen Gewinnung von Dimethylether aus Methanol mit $Al_2O_3$ - bzw. $Al_2O_3/SiO_2$-Katalysatoren im geraden Durchgang am Ausgang des Synthesereaktors anfallen.

Bei Dimethylethergehalten, die zwischen > 5 und < 20 Gew.-% liegen bzw. oberhalb 80 Gew.-% liegen, sind die erfindungsgemäßen Rückflußverhältnisse auf der Bais der angegebenen Werte zu wählen wie z.B. für Gehalte von > 5 - < 20 Gew.-% zwischen einem Rückflußverhältnis von 1:0,2 bis 1:50, bevorzugt von 1:0,4 bis 1:25 zu wählen bzw.

bei > 80 Gew.-% auch bei einem Verhältnis 1:0,2 bis 1:50, bevorzugt von 1:0,2 bis 1:10 und besonders bevorzugt von 1:0,4 bis 1:5.

Ein reiner Dimethylether kann auch dadurch erhalten werden, daß man ohne Waschflüssigkeit bei den in Anspruch 2 angegebenen Rückflußverhältnissen arbeitet.

Die Destillationskolonne zur Gewinnung des reinen Dimethylethers wird im allgemeinen bei einem Druck von 5 - 1o bar betrieben, wobei im Falle eines vorgeschalteten Synthesereaktors bevorzugt eine Anpassung an den Druck im Reaktor erfolgt, wobei im allgemeinen die Destillation bei einem etwas niedrigeren Druck als die Synthese betrieben wird. Drucke außerhalb dieses Bereichs sind erfindungsgemäß ebenfalls möglich.
Der Durchsatz wird wie üblich, durch die Auslegung der Kolonne, die Wärmezufuhr und das Rückflußverhältnis bestimmt.

Zur nahezu quantitativen Gewinnung des reinen Dimethylethers, insbesondere bei kleinen Gehalten des Dimethylethers in Einsatzprodukt kann erfindungsgemäß das über Kopf der Kolonne gehende Abgasgemisch, das im allgemeinen $CO_2$, $N_2$, Kohlenwasserstoffe und noch kleine Anteile an Dimethylether enthält, gewaschen werden. Die den Dimethylether enthaltende Waschflüssigkeit kann in die Kolonne oder in den Dimethylether-Synthesereaktor rückgeführt werden.

Geeignete Waschflüssigkeiten sind beispielsweise Methanol oder Sumpfprodukte der Dimethylether-Destillationskolonne. Das Waschen kann im Gleich- oder Gegenstrom erfolgen, bevorzugt jedoch im Gegenstrom.

Ferner kann das die Verunreinigungen enthaltende Destillat zusätzlich in einem Seitenstripper gestrippt werden, wobei das Stripp-Produkt wieder in die Kolonne zurückgeführt wird. Hierdurch kann ggfs. an dieser Stelle ausgetretener Dimethylether nahezu vollständig rückgeführt werden.

Wie für den Fachmann deutlich ist, wird erfindungsgemäß das bei 64,7°C siedende Methanol von Wasser in einer 2. Kolonne, die als Abtreibkolonne betrieben wird, abgetrennt.

Nach dem erfindungsgemäßen Verfahren können Destillationskolonnen nach dem Stand der Technik entsprechend der Anlagenkapazität eingesetzt werden.

Die Böden können die dem Stand der Technik entsprechenden Böden sein, wie beispielsweise Ventilböden, Siebböden, Glockenböden u.a.

Grundsätzlich können auch Füllkörper oder Pakungen wie beispielsweise keramsiche Materialien, Glasmaterialien, Packungen aus Draht und dergleichen als Füllung für die Destillationskolonne verwendet werden, wobei die erfindungsgemäße Einsatzproduktzuführung, bzw. die Entnahmestellen für Dimethylether und Vorlauf gemäß den erforderlichen Bodenzahlen errechnet werden können auf der Grundlage der in der vorliegenden Erfindung angegebenen praktischen Böden.

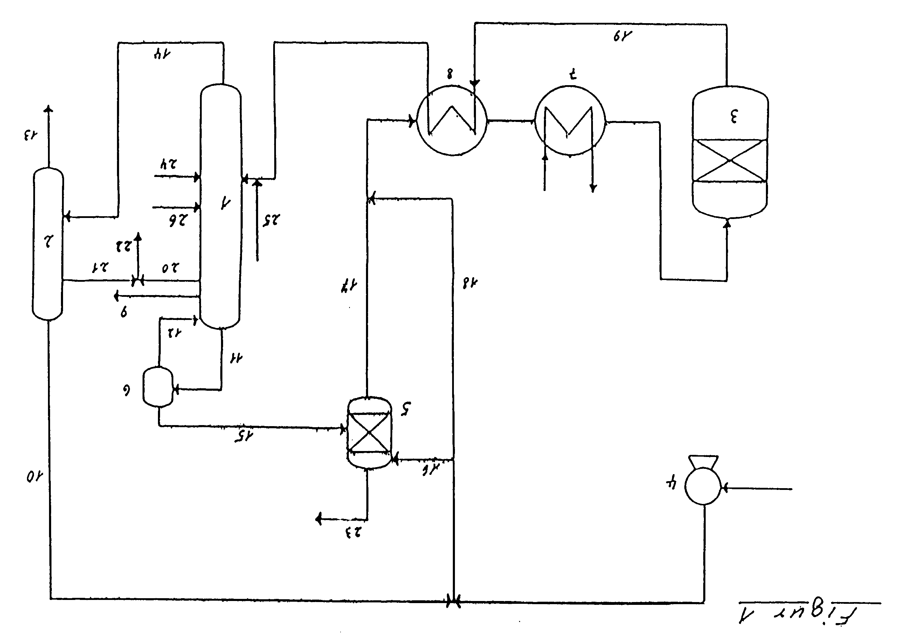

Die Erfindung wird beispielhaft mit Hilfe der Figuren 1 bis 3 näher erläutert.

In Figur 1 stellt (3) den Dehydratisierungsreaktor dar. Über Pumpe (4), Leitung (18) und Wärmetauscher (8) und (7) wird frisches Methanol dem Reaktor zugeführt. (1) stellt die Destillationskolonne zur Gewinnung des reinen Dimethylethers dar. Die Kolonne wird im allgemeinen in einem Druckbereich betrieben, der dem Druck in Synthesereaktor (3) angepaßt ist, üblicherweise bei etwas niedrigerem Druck. Liegt beispielsweise der Synthesedruck bei 8 - 12 bar, so kann die Kolonne beispielsweise bei 6 - 11,5 bar betrieben werden. Diese Zahlen sind jedoch nicht als einschränkend anzusehen.
Im allgemeinen wird jedoch bevorzugt bei einer Druckdifferenz von 0 bis zu 10 bar gearbeitet. Der Dimethylether wird bei (9) in hochreiner Form abgezogen. Kopfgas gelangt über (11) in Kondensator (6). Rückfluß fließt über (12) wieder auf Kolonne (1). Das Abgas fließt über (15) in Wäscher (5), in dem es mit Methanol (16) gewaschen wird. Grundsätzlich können auch andere Waschflüssigkeiten eingesetzt werden, wie beispielsweise Rohmethanol oder Sumpf der Kolonne 1, wobei im letzteren Fall die den Dimethylether enthaltende Waschflüssigkeit wieder der Kolonne 1 zugeführt werden kann. Kleine Mengen Dimethylether gelangen über (17) zusammen mit Waschmethanol in den Synthesereaktor (3). Das Abgas entweicht bei (23). Das Syntheseprodukt (19) fließt über (8) nach (1). Bei (2o) werden die zwischen Methanol und Dimethylether siedenden Verunreinigungen abgezogen und gelangen über (22) beispielsweise zur Verbrennung. Über die beispielhaften Zuführungen (24), (25) und/oder (26) wird erfindungsgemäß Waschflüssigkeit zugeführt.

Über (14) fließt der im wesentlichen Methanol und Wasser enthaltende Sumpf in Abtreibkolonne (2). Diese wird im allgemeinen bei Normaldruck betrieben, kann jedoch prinzipiell auch bei etwas erhöhtem oder etwas niedrigerem Druck betrieben werden, jedoch im allgemeinen unterhalb des Druckes der Kolonne (1). Über (10) wird Methanol abgezogen und zum Synthesereaktor zurückgeführt. Aus dem Sumpf wird über (13) Abwasser abgezogen. Über (21) können im Bedarfsfall Verunreinigungen entnommen werden mit höheren Siedepunkten als dem des Methanols. Das Methanol kann auch an einem der oberen Böden der Kolonne (2) als Seitenabzug entnommen werden. Kleine Mengen an Verunreinigungen können dann noch über Kopf entnommen werden.

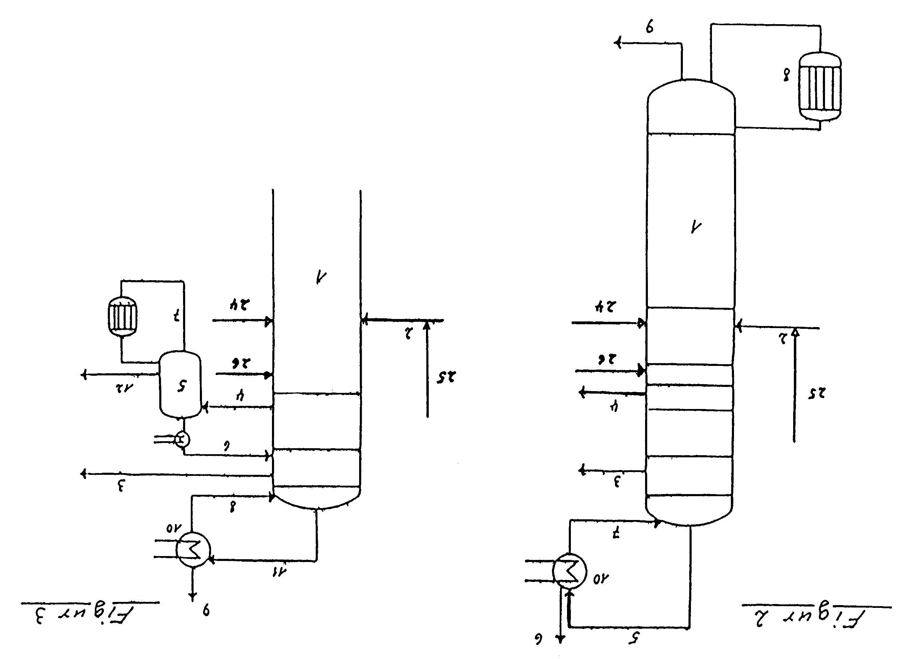

In Figur 2 stellt (1) die Destillationskolonne zur Gewinnung von reinem Dimethylether dar. Das Einsatzprodukt wird über (2) zugeführt. Der reine Dimethylether wird bei (3) entnommen. Die Verunreinigungen werden bei (4) abgezogen. Das Kopfprodukt fließt über (5) zum Kondensator (1o). Rückfluß fließt über (7) zu Kolonne (1). Abgas entweicht bei (6). (8) stellt den Reboiler-Kreislauf dar. Bei (9) wird der Sumpf abgezogen. Über (24), (25) und (26) wird erfindungsgemäß Waschflüssigkeit zugeführt.

In Figur 3 fließt das die Verunreinigungen enthaltende Destillat über (4) zum Stripper (5), der mit Reboiler-Kreislauf (7) ausgerüstet ist. Über (24), (25) und (26) wird erfindungsgemäß Waschflüssigkeit zugeführt.

Abgestrippter Dimethylether fließt über (6) zur Kolonne (1) zurück.

Bei (2) wird Einsatzprodukt zugeführt. Bei (3) wird reiner Dimethylether entnommen, Kopfgas gelangt über (11) in Kondensator (1o). Rückfluß fließt über (8) zur Kolonne (1). Abgas entweicht bei (9). Bei (12) werden die von Dimethylether befreiten Verunreinigungen abgenommen.

Die Waschflüssigkeit kann auch in Dampfform oder teilweise in Dampfform und teilweise flüssig in die Kolonne eingeführt werden.

Gemäß vorliegender Erfindung wird aus der Destillationskolonne der hochreine Dimethylether praktisch quantitativ gewonnen. Der Dimethylether ist geruchsfrei, enthält weniger als 10 ppm Methanol, maximal o,1 Gewichtsprozent Kohlenwasserstoffe und besitzt eine Reinheit von wenigstens ca. 99,9 Gew.%. Dimethylether. Der erfindungsgemäß gewonnene Dimethylether ist für jede Anwendung im Aerosolbereich hervorragend geeignet.

Beispiel 1

Einer Kolonne mit 65 Ventilböden wurden am 48. Boden (vom Kopf der Kolonne) 4493 kg/h eines Dehydratisierungsproduktes zugeführt, das aus 26oo kg Dimethylether, 72o kg Methanol, 1o6o kg Wasser und 113 kg Verunreinigungen bestand und in Gegenwart eines erfindungsgemäßen Katalysators hergestellt wurde sowie 2ooo kg/h Reinmethanol über die gleiche Zuführungsleitung (Reinheit: 99,9 Gew.-%).

Die Kolonne wurde bei 8 bar betrieben.

Das Rückflußverhältnis lag bei 1:2,4.

Am Kopf der Kolonne wurden ca. 16 kg/h Abgas abgezogen, im wesentlichen bestehend aus $CO_2$, $N_2$, Kohlenwasserstoffen und kleinen Anteilen an Dimethylether.

Am 5. Boden der Kolonne (vom Kolonnenkopf) wurden 2595 kg/h reiner, geruchsfreier Dimethylether abgezogen mit einem Methanolgehalt von 9 ppm und einem Gehalt an Kohlenwasserstoffen von 130 ppm.

Am 22. Boden (vom Kolonnenkopf) wurden 92 kg/h an Verunreinigungen abgezogen. 12 kg/h an höher siedenden Komponenten (als Methanol), 2718 kg/h Methanol und 1o6o kg/h Wasser wurden am Sumpf abgezogen und einer Kolonne zur Methanolrückführung zugeführt.

Vergleichsbeispiel 1a

Beispiel 1 wurde wiederholt, jedoch ohne Zuführung von zusätzlichem Methanol. Das Rückflußverhältnis betrug 1:0,8.

Es wurden 26 kg/h Gase am Kopf der Kolonne abgezogen.

Am 5. Boden wurden 2565 kg/h eines reinen, geruchsfreien Dimethylethers abgezogen mit einem Methanolgehalt von 9 ppm und einem Kohlenwasserstoffgehalt von 150 ppm.

Am 22. Boden wurden 91 kg/h an Verunreinigungen abgezogen.

11 kg/h an höher siedenden Verunreinigungen, 72o kg/h Methanol und 1o6o kg/h Wasser wurden am Sumpf abgezogen.

Beispiel 2

In die gleiche Kolonne wie in Beispiel 1 wurde das gleiche Einsatzprodukt eingesetzt.

Die Kolonne wurde wie in Beispiel 1 betrieben, jedoch wurden die Verunreinigungen am 46. Boden (vom Kolonnenkopf) abgezogen.

Der erhaltene reine Dimethylether war nahezu geruchsfrei und enthielt 8 ppm Methanol und einen Gehalt an Kohlenwasserstoffen von 136 ppm.

Vergleichsbeispiel 2a

Beispiel 2 wurde wiederholt, jedoch ohne Zuführung, von zusätzlichem Methanol.

Der erhaltene Dimethylether hatte einen schwachen Geruch, enthielt 8 ppm Methanol und 205 ppm Kohlenwasserstoffe.

Beispiel 3

Einer Destillationskolonne mit 1oo Ventilböden wurden pro Stunde 66.000 kg eines Dehydratisierungsgemischs zugeführt, das aus 62.000 kg Methanol, 25oo kg Dimethylether, 98o kg Wasser und 52o kg Verunreinigungen bestand.

Die Kolonne wurde bei 7,5 bar betrieben.

Das Rückflußverhältnis betrug 1:10.

Zusätzlich wurden 1o.ooo kg/h Rohmethanol am 35. Boden (vom Kolonnenkopf) zugeführt.

Das Rohmethanol stammte aus einer Lurgi-Niederdruckmethanolanlage und enthielt 93 Gew.-% Methanol, o.5 Gew.-% Kohlenwasserstoffe und 6,5 Gew.-% Wasser.

Das Dehydratisierungsprodukt wurde am 48. Boden (vom Kolonnenkopf) zugeführt.

298 kg/h Verunreinigungen wurden am 42. Boden (vom Kopf der Kolonne) abgezogen.

2495 kg/h reiner Diemthylether wurden am 4. Boden (vom Kolonnenkopf) abgezogen.

Über Kopf wurden 23 kg/h Abgas abgezogen.

Aus dem Sumpf wurden 244kg/h höher siedende Verunreinigungen, 71.3000 kg/h Methanol und 163o kg/h Wasser abgezogen.

Der Dimethylether enthielt 6 ppm Methanol, 220 ppm Kohlenwasserstoff und war völlig geruchlos.

Vergleichsbeispiel 3a

Beispiel 3 wurde wiederholt, jedoch ohne Rohmethanolzufuhr. Das Rückflußverhältnis betrug 1:7. Es wurden 2487 kg/h geruchloser Dimethylether erhalten, der 250 ppm Kohlenwasserstoffe und 5 ppm Methanol enthielt.

Beispiel 4

Einer Destillationskolonne mit 5o Ventilböden wurden 448o kg/h eines Gemisches zugeführt, das aus 26oo kg Dimethylether, 62o kg Methanol, 1o2o kg Wasser und 24o kg Verunreinigungen bestand. Zusätzlich wurden auf den Einspritzboden für das Dehydratisierungsprodukt 15oo kg/h Reinmethanol gefahren.

Das Dehydratisierungsprodukt wurde in Gegenwart eines $\gamma$-$Al_2O_3$ Katalysators erzeugt, der 6 Gew.-% $SiO_2$ enthielt. Es wurde bei einem Druck von 1o bar und einem Rückflußverhältnis von 1:2 gearbeitet.

2592 kg/h reiner Dimethylether wurden am 4. Boden (vom Kolonnenkopf) abgezogen.

Das Dehydratisierungsprodukt wurde am 4o. Boden (vom Kopf der Kolonne) eingesetzt.

211,5 kg/h an Verunreinigungen wurden am 25. Boden abgezogen. Über Kopf wurden 18kg/h an Gasen abgezogen.

Aus dem Sumpf wurden 2118,5 kg/h Methanol, 20 kg/h höher siedende Verunreinigungen und 1020 kg/h Wasser abgezogen. Es wurde ein geruchsfreier Dimethylether erhalten, der 3 ppm Methanol und 125 ppm Kohlenwasserstoffe enthielt.

Beispiel 5

Einer Kolonne mit 70 Glockenböden wurden am 48. Boden (vom Kopf der Kolonne) 4000 kg/h eines Gemisches zugeführt, das aus 800 kg Dimethylether , 2825 kg Methanol, 310 kg Wasser und 65,5 kg Verunreinigungen bestand.

Es wurde bei einem Kolonnendruck von 8 bar und einem Rückflußverhältnis von 1:4 gearbeitet. Zusätzlich wurden 5oo kg/h Reinmethanol am 38. Boden zugeführt. Am 17. Boden (vom Kolonnenkopf) wurden 798 kg/h Dimethylether abgenommen.

Am 35. Boden wurden 55 kg/h an Verunreinigungen abgezogen.

Aus dem Sumpf wurden 3324,5 kg/h Methanol, 310 kg/h Wasser und 7,5 kg/h an höher siedenden Verunreinigungen abgezogen. Über Kopf wurden 5 kg/h Abgas abgezogen.

Es wurde ein Dimethylether mit schwachem Geruch erhalten mit 810 mg Kohlenwasserstoffen und 8 mg Methanol.

Vergleichsbeispiel 5a

Beispiel 5 wurde wiederholt, jedoch ohne Zusatz von Methanol. Der Dimethylether, der in einer Menge von 791 kg/h erhalten wurde, hatte einen deutlich stärkeren Geruch als der gemäß Beispiel 5 erhaltene.

Der Kohlenwasserstoffanteil lag bei 870 mg, die Methanolkonzentration bei 8 mg.

Beispiel 6

In eine Glockenbodenkolonne mit 100 Böden wurde am 33. und 46. Boden ein Gemisch von 40.000 kg/h Rohmethanol und 3500 kg/h Dehydratisierungsprodukt eingesetzt.

Das Rohmethanol entsprach der in Beispiel 3 eingesetzten Qualität und wurde direkt aus dem Druckabscheider der Methanolysnthese entommen. Das Dehydratisierungsprodukt bestand aus 2200 kg Dimethylether, 861 kg Wasser, 385 kg Methanol und 54 kg Verunreinigungen. Es wurde bei 6,5 bar und einem Rückflußverhältnis von 1:12 gefahren.

Am 6. Boden der Kolonne (vom Kopf der Kolonne) wurden 2199 kg/h reiner Dimethylether abgezogen. Über kopf gingen 54 kg/h Abgas.

Am 26. Boden wurden 85 kg/h Verunreinigungen abgezogen.

Am Sumpf wurden 3461 kg/h Wasser, 3758,5 kg/h Methanol und 116 kg/h an höher siedenden Verunreinigungen abgezogen.

Es wurde ein völlig geruchsfreier Dimethylether erhalten, der 90 ppm Kohlenwasserstoffe und 7 ppm Methanol enthielt.

Beispiel 7

Beispiel 1 wurde wiederholt, jedoch wurden anstelle von Rohmethanol, 2ooo kg/h Wasser zugesetzt.

Es wurde ein reiner, geruchsfreier Dimethylether erhalten, der 4 ppm Methanol und 105 ppm Kohlenwasserstoffe enthielt.

Ähnliche Ergebnisse wurden mit Methanol/Wassergemischen erhalten.

### Beispiel 8

Die Aufarbeitung des Dehydratisierungsproduktes (3ooo kg/h Einsatzprodukt mit 65 Gew.-% Dimethylether) erfolgte in 2 hintereinander geschalteten kontinuierlich arbeitenden Destillationskolonnen, von denen die erste mit 5o Ventilböden und die 2. mit Raschigringen ausgerüstet war. Der reine Dimethylether wurde in der ersten Kolonne bei 7 bar am 4. Boden (vom Kopf der Kolonne) bei einem Rückflußverhältnis von 1:2 gewonnen. Das Einsatzprodukt wurde am 27. Boden (vom Kopf der Kolonne) zugeführt. Die Verunreinigungen wurden am 22. Boden abgezogen. In der 2. Kolonne wurde nicht umgesetztes Methanol zurückgewonnen.
Es wurde ein geruchsfreier Dimethylether erhalten.

### Vergleichsbeispiel 8a

Es wurde die in Beispiel 8 gearbeitet, jedoch die Verunreinigungen am Kopf der 2. Kolonne abgezogen.
Der so gewonnene Dimethylether hatte einen deutlichen durch Verunreinigungen bedingten Geruch.

### Vergleichsbeispiel 8b

Es wurde wie in Beispiel 8a gearbeitet, zusätzlich wurden jedoch 3000 kg/h Rohmethanol gemeinsam mit dem Dehydratisierungsprodukt in die Kolonne eingesetzt.
Der Dimethylether besaß nur noch einen schwachen Geruch.

### Beispiel 9

Vergleichsbeispiel 1a wurde wiederholt, jedoch enthielt das eingesetzte Dehydratisierungsprodukt 1124 kg Dimethylether, 2030 kg Methanol, 1280 kg Wasser und 59 kg an Verunreinigungen.
Das Rückflußverhältnis betrug 1:30.
Es wurden 1120 kg/h eines hochreinen, geruchsfreien Dimethylethers erhalten mit 4 ppm Methanol und 90 ppm Kohlenwasserstoffen.

### Beispiel 10

Vergleichsbeispiel 1a wurde wiederholt, jeoch enthielten 4000 kg Dehydratisierungsgemisch 2850 kg Dimethylether, 780 kg Methanol, 335 kg Wasser und 35 kg Verunreinigungen. Das Rückflußverhältnis betrug 1:26.
Es wurden 2843 kg/h eines sehr reinen geruchsfreien Dimethylethers erhalten mit 3 ppm Methanol und 76 ppm Kohlenwasserstoffen.
Bei einem Rückflußverhältnis von 1:0,3 wurden 2773 kg/h geruchsfreier Dimethylether erhalten, der 8 ppm Methanol und740 ppm Kohlenwasserstoffe enthielt.

Beispiel 1 und Vergleichsbeispiel 1a zeigen, daß sich durch zusätzliches Methanol die Menge an gewinnbarem reinen Dimethylether erhöht.

Aus Beispiel 2 und Vergleichsbeispiel 2a geht hervor, daß auch in einem Fall, in dem die Verunreinigungen nicht um mindestens 3 Böden oberhalb des Zuführungsbodens abgezogen werden, durch Zusatz von Waschflüssigkeit ein nahezu geruchsfreier Dimethylether erhalten wird.

Demgemäß kann die Waschflüssigkeit und deren eingesetzte Menge als Regulativ im Falle von Abweichungen von der in Anspruch 1 offenbarten Fahrweise angewandt werden. Die Verwendung von Waschflüssigkeit kann erfindungsgemäß darüber hinaus ganz allgemein in Destillationen, in denen reiner Dimethylether gewonnen wird, zur Verbesserung der Dimethyletherqualität und der Erhöhung der abdestillierbaren Menge erfolgen, wie Beispiel 8 zeigt.

Aus Beispiel 3 und Vergleichsbeispiel 3a geht hervor, daß auch bei Einsatz von Rohmethanol als Waschflüssigkeit eine erhöhte Menge an reinem Dimethylether gewonnen werden kann.

Beispiel 4 und Vergleichsbeispiel 4a zeigen, daß auch bei Verwendung eines relativ ungünstigen $\gamma$-$Al_2O_3$ Katalysators mit einem vergleichsweise hohen $SiO_2$-Gehalt, durch Einsatz von Methanol als Waschflüssigkeit, ein Dimethylether mit verbesserter Qualität gewonnen werden kann.

Beispiel 5 und Vergleichsbeispiel 5a zeigen, daß auch bei Abziehen des reinen Dimethylethers am 17. Boden (vom Kopf der Kolonne) durch Zusatz von Waschflüssigkeit die Qualität des Dimethylethers, insbesondere bezüglich des Geruchs, verbessert werden kann.

In Beispiel 6 ist eine typische integrierte Destillation von Rohmethanol aus einer Methanolsyntheseanlage und Dehydratisierungsprodukt aus einer Dimethylether-Syntheseanlage offenbart. Das Verhältnis der beiden Produkte ist ca. 11:1, es kann jedoch, wie oben ausgeführt, in Abhängigkeit von der Kapazität der Anlagen stark variieren.
Das Beispiel zeigt, daß ein hochreiner Dimethylether bei einer solchen Fahrweise gewonnen werden kann, ohne daß die Gewinnung eines spezifikationsgerechten Reinmethanols beeinträchtigt wird (im Beispiel nicht offenbart).

Aus Beispiel 7 geht hervor, daß auch mit Wasser und Methanol/Wasser-Gemischen die Qualität des Dimethylethers verbessert werden kann.

Schließlich zeigen die Beispiel 8 und die Vergleichsbeispiele 8a und 8b, daß auch bei Abweichung von der erfindungsgemäßen Destillationsmethode, die Verwendung von Waschflüssigkeit die Qualität des Dimethylethers verbessert.

Die Beispiel 9 und 10 zeigen, daß im Konzentrationsbereich von 20 - 80 Gew.-% Dimethylether insbesondere bei hohen Rückflußverhältnissen ein hochreiner Dimethylether in hohen Ausbeuten erhalten wird. Auch bei niederen Rückflußverhältnissen und insbesondere hohem Dimethyletheranteil wird eingeruchsfreier Dimethylether in relative hoher Ausbeute erhalten.

**Patentansprüche**

1. Verbessertes Verfahren zur Herstellung von reinem Dimethylether durch katalytische Dehydratisierung von Methanol bei einer Temperatur von 140 - 500 °C und einem Druck von 1 bis 50 bar und destillative Aufarbeitung des Dehydratisierungsprodukts dadurch gekennzeichnet, daß

a, das Dehydratisierungsprodukt in die Kolonne zur Gewinnung von reinem Dimethylether unterhalb des 25. Bodens (vom Kopf der Kolonne) an einem oder mehreren Böden in die Kolonne und mindestens 1 Boden oberhalb des Kolonnensumpfes zugeführt wird,

b, der reine Dimethylether ab dem 15. Boden, bevorzugt 10. Boden (vom Kopf der Kolonne) an einem Boden oder mehreren Böden abgezogen wird,

c, daß ein Verunreinigungen, die höher als Dimethylether und niedriger als Methanol sieden, enthaltendes Destillat an einem Boden oder mehreren Böden abgezogen wird, der (die) um mindestens 3 Böden oberhalb des (obersten) Zulaufs des den Dimethylether enthaltenden Einsatzgemisches und um mindestens einen Boden unterhalb des 15. Bodens (vom Kopf der Kolonne) liegt (liegen), wobei der Abstand zwischen dem Abzugsboden für reinen Dimethylether und Verunreinigungen bevorzugt wenigstens 5 Böden beträgt,

d, zusätzlich zum Dehydratisierungsprodukt aus dem Dimethylether-Synthesereaktor, eine Waschflüssigkeit eingesetzt wird,

wobei bei eimen Gesamt-Einsatzprodukt, einschließlich Waschflüssigkeitszusatz gemäß d), das > 0 - 5 Gew.-% Dimethylether enthält, ein Rückflußverhältnis von 1:0,2 bis 1:100, bevorzugt 1:1 bis 1:25 gefahren wird, bei einem Gesamt-Einsatzprodukt, das 20 - 80 Gew.-% Dimethylether enthält, ein Rückflußverhältnis von 1:0,2 bis 1:50 gefahren wird, bevorzugt von 1:0,5 bis 1:10 und besonders bevorzugt von 1:1 bis 1:5, bei einem Gesamt-Einsatzprodukt, das >5 - <20 Gew.-% Dimethylether enthält, ein Rückflußverhältnis von 1:0,2 bis 1:50, bevorzugt von 1:0,4 bis 1:25, und bei einem Gesamt-Einsatzprodukt, das >80 Gew.-% und <99 Gew.-% Dimethylether enthält, ein Rückflußverhältnis von 1:0,2 bis 1:50, bevorzugt von 1:0,2 bis 1:10 und besonders bevorzugt von 1:0,4 bis 1:5 gefahren wird.

2. Verfahren zur Herstellung von reinem Dimethylether durch katalytische Dehydratisierung von Methanol bei einer Temperatur von 140 - 500 °C und einem Druck von 1 bis 50 bar und destillative Aufarbeitung des Dehydratisierungsprodukts, wobei das Dehydratisierungsprodukt in die Kolonne, zur Gewinnung von reinem Dimethylether unterhalb des 25. Bodens (vom Kopf der Kolonne) an einem oder mehreren Böden in die Kolonne eingesetzt wird und das Dehydratisierungsprodukt mindestens 1 Boden oberhalb des Kolonnensumpfes zugeführt wird, der reine Dimethylether ab dem 15. Boden, bevorzugt ab dem 10. Boden (vom Kopf der Kolonne) an einem Boden oder mehreren Böden abgezogen wird, ein Verunreinigungen, die höher als Dimethylether und niedriger als Methanol sieden, enthaltendes Destillat an einem Boden oder mehreren Böden abgezogen wird, der (die) um mindestens 3 Böden oberhalb des (obersten) Zulaufs des den Dimethylether enthaltenden Einsatzgemisches und um mindestens einen Boden unterhalb des 15. Bodens, (vom Kopf der Kolonne) liegt (liegen), dadurch gekennzeichnet, daß bei einem Einsatzprodukt, das > 0 - 5 Gew.-% Dimethylether enthält, ein Rückflußverhältnis von 1:0,2 bis 1: < 1 gefahren wird, bei einem Einsatzprodukt, das 20 - 80 Gew.-% Dimethylether enthält, ein Rückflußverhältnis von 1:0,2 bis 1: < 0,4 oder von 1: > 5 bis 1: 50 gefahren wird, und bei einem Einsatzprodukt, das >5 - <20 Gew.-% Dimethylether enthält, ein Rückflußverhältnis 1:0,2 bis 1:<0,4 oder 1:>25 bis 1:50 gefahren wird. bzw. bei > 80 Gew.-% bis 99 Gew.-% Dimethylether 1:>5 bis 1:50.

3. Verfahren nach Anspruch 1, dadurch gekennzeichnet, daß die Waschflüssigkeit Methanol oder ein Methanol/Wassergemisch ist.

4. Verfahren nach Anspruch 1, dadurch gekennzeichnet, daß die Waschflüssigkeit Wasser ist.

5. Verfahren nach den Ansprüchen 1 - 4, dadurch gekennzeichnet, daß mit einem Katalysator vom $\gamma$-Al$_2$O$_3$-Typ, der > 0 - 1 Gew.-% SiO$_2$ enthält, dehydratisiert wird.

6. Verfahren nach den Ansprüchen 1 - 5, dadurch gekennzeichnet, daß mit einem Katalysator vom $\gamma$-Al$_2$O$_3$-Typ, der 0,001 bis 0,5 Gew.-% SiO$_2$ enthält, dehydratisiert wird.

7. Verfahren nach den Ansprüchen 1 - 6, dadurch gekennzeichnet, daß mit einem Katalysator vom $\gamma$-Al$_2$O$_3$-Typ, der 0,001 bis 0,2 Gew.-% SiO$_2$ enthält, dehydratisiert wird.

8. Verfahren nach den Ansprüchen 1 - 7, dadurch gekennzeichnet, daß bei einer Temperatur von 140° bis 500 °C, bevorzugt von 140° bis 450 °C und bei einem Druck von 1 bar bis 50, bevorzugt von 3 - 25 bar dehydratisiert wird.

9. Verfahren nach den Ansprüchen 1 - 8, dadurch gekennzeichnet, daß bei einer Kontaktbelastung (LHSV) von 0,2 - 16 l/l h, dehydratisiert wird.

10. Verfahren nach den Ansprüchen 1 - 9, dadurch gekennzeichnet, daß bei einer Kontaktbelastung (LHSV) von 0,5 - 13,5 l/l h dehydratisiert wird.

11. Verfahren nach den Ansprüchen 1 - 10, dadurch gekennzeichnet, daß die am Kopf der Destillationskolonne zur Gewinnung reinen Dimethylethers abgezogenen leichten Komponenten, die im allgemeinen CO$_2$, N$_2$, leichte Kohlenwasserstoffe auch Dimethylether enthalten, in Gegen- oder Gleichstrom gewaschen werden, vorzugsweise mit Methanol.

12. Verfahren nach den Ansprüchen 1 - 11, dadurch gekennzeichnet, daß die unterhalb des Abzugs des reinen Dimethylethers abgezogenen Verunreinigungen in einem Seitenstripper gestrippt werden und daß der so abgetrennte Dimethylether in die Destillationskolonne rückgeführt wird.

13. Verfahren nach den Ansprüchen 1 - 12, dadurch gekennzeichnet, daß das Dehydratisierungsprodukt unterhalb des 25. Bodens an einem oder mehreren Böden je nach Bodenzahl zwischen dem 50. und 25. Boden in die Kolonne eingesetzt wird.

14. Verfahren nach den Ansprüchen 1 - 13, dadurch gekennzeichnet, daß das Dehydratisierungsprodukt mindestens 5 Böden oberhalb des Kolonnensumpfes zugeführt wird.

15. Verfahren nach den Ansprüchen 1 - 14, dadurch gekennzeichnet, daß das Dehydratisierungsprodukt mindestens 10 Böden oberhalb des Kolonnensumpfes zugeführt wird.

16. Verfahren nach den Ansprüchen 1 und 3 - 15, daduch gekennzeichnet, daß die Waschflüssigkeit in die Destillationskolonne zwischen dem einschließlich 3. Boden unterhalb des Abzugsbodens für reinem Dimethylether und dem 3. Boden von unten, eingesetzt wird.

17. Verfahren nach den Ansprüchen 1 und 3 - 16, dadurch gekennzeichnet, daß die Waschflüssigkeit gemäß Anspruch 1 (d) unterhalb des untersten Zuführungsbodens für das Dehydratisierungsprodukt zugeführt wird.

18. Verfahren nach den Ansprüchen 1 und 3 - 17, dadurch gekennzeichnet, daß das Dehydratisierungsprodukt aus dem Dimethylethersynthesereaktor und die Waschflüssigkeit vor Einsatz in die Dimethyletherkolonne zusammengeführt werden.

19. Verfahren nach den Ansprüche 1 und 3 - 18, dadurch gekennzeichnet, daß das Dehydratisierungsprodukt aus dem Dimethylethersynthesereaktor und die Waschflüssigkeit getrennt, jedoch auf den (die) gleichen Einspritzboden aufgegeben wird (werden).

20. Verfahren nach den Ansprüchen 1 und 3 - 19, dadurch gekennzeichnet, daß das als Waschflüssigkeit eingesetzte Methanol zumindest teilweise Rohmethanol aus einer Methanolsynthese ist.

**Claims**

1. An improved process for producing pure dimethyl ether by catalytic dehydration of methanol at temperatures of between 140 and 500 °C and pressures of between 1 and 50 bar and by purifying distillation of the dehydration product, which comprises:

 (a) feeding the dehydration product to one plate or several plates below the 25th plate[*] of a column for producing pure DME, at least one plate above the bottom of said column,

 (b) withdrawing the pure DME from one plate or several plates from the 15th plate of the column, preferably from the 10th,

 (c) withdrawing the distillate containing impurities with higher boiling points than DME and lower boiling points than methanol from one plate or several plates located at least three plates above the highest feed point for the DME-containing mixture and at least

[*] The numbers of the plates referred to in this patent application are meant from the top of the column unless otherwise specified.

one plate below the 15<sup>th</sup> plate, the space between the plates for removing DME and impurities being preferably at least five plates,

(d) charging a washing liquid in addition to the dehydration product from the DME synthesis reactor,

wherein the reflux ratio is 1:0.2 to 1:100, preferably 1:1 to 1:25, if the feedstock, including the washing liquid herein referred to d), contains >0 to 5 weight percent DME, the reflux ratio is 1:0.2 to 1:50, preferably 1:0.5 to 1:10, most preferably 1:1 to 1:5, if the feedstock contains 20 to 80 weight percent DME, the reflux ratio is 1:0.2 to 1:50, preferably 1:0.4 to 1:25, if the feedstock contains >5 weight percent to <20 weight percent DME, the reflux ratio is 1:0.2 to 1:50, preferably 1:0.2 to 1:10, most preferably 1:0.4 to 1:5, if the feedstock contains >80 weight percent to <99 weight percent DME.

2. A process for producing pure dimethyl ether by catalytic dehydration of methanol at temperatures of between 140 and 500 °C and pressures of between 1 and 50 bar and purifying distillation of the dehydration product, which comprises that the dehydration product is fed to one plate or several plates below the 25th plate of the column for producing pure dimethyl ether, at least one plate above the bottom of said column, the pure DME is withdrawn from one plate or several plates from the 15<sup>th</sup> plate, preferably from the 10<sup>th</sup>, the distillate which contains impurities with higher boiling points than DME and lower boiling points than methanol is withdrawn from one plate or several plates located at least three plates above the top feed plate for the DME-containing mixture and at least one plate below the 15<sup>th</sup>, wherein the reflux ratio is 1:0.2 to 1:<1 if the feedstock contains >0 to 5 weight percent DME, the reflux ratio is 1:0.2 to 1:<0.4 or 1:>5 to 1:50 if the feedstock contains 20 to 80 weight percent DME, the reflux ratio is 1:0.2 to 1:<0.4 or 1:>25 to 1:50 if the feedstock contains >5 to <20 weight percent DME, and the reflux ratio is 1:>5 to 1:50 if the feedstock contains >80 to 99 weight percent dimethyl ether.

3. A process in accordance with claim 1 wherein the washing liquid is methanol or a methanol/water mixture.

4. A process in accordance with claim 1 wherein the washing liquid is water.

5. A process as claimed in any of the claims 1 to 4 wherein a catalyst of the $\gamma$-$Al_2O_3$ type containing >0 to 1 weight percent $SiO_2$ is used for the dehydration.

6. A process as claimed in any of the claims 1 to 5 wherein a catalyst of the $\gamma$-$Al_2O_3$ type containing 0.001 to 0.5 weight percent $SiO_2$ is used for the dehydration.

7. A process as claimed in any of the claims 1 to 6 wherein a catalyst of the $\gamma$-$Al_2O_3$ type containing 0.001 to 0.2 weight percent $SiO_2$ is used for the dehydration.

8. A process as claimed in any of the claims 1 to 7 wherein the dehydration is carried out at a temperature in the range of between 140 °C and 500 °C, preferably between 140 °c and 450 °C, and at a pressure in the range of between 1 and 50 bar, preferably 3 and 25 bar.

9. A process as claimed in any of the claims 1 to 8 wherein the LHSV during dehydration is from 0.2 to 16 litres/litre and hour.

10. A process as claimed in any of the claims 1 to 9 wherein the LHSV during dehydration is from 0.5 to 13.5 litres/litre and hour.

11. A process as claimed in any of the claims 1 to 10 wherein the light components removed at the head of the column for producing pure dimethyl ether, said components generally containing $CO_2$, $N_2$, light hydrocarbons as well as dimethyl ether, are washed in co-current or countercurrent operation, preferably with methanol.

12. A process as claimed in any of the claims 1 to 11 wherein the impurities removed below the withdrawal plate for pure dimethyl ether are charged to a lateral stripping column in order to separate the dimethyl ether which is then recycled to the distillation column.

13. A process as claimed in any of the claims 1 to 12 wherein the dehydration product is fed below the 25<sup>th</sup> plate of the column to one or several plates from the 25<sup>th</sup> plate to the 50<sup>th</sup>.

14. A process as claimed in any of the claims 1 to 13 wherein the dehydration product is charged to the column at a point which is at least five plates above its bottom.

15. A process as claimed in any of the claims 1 to 14 wherein the dehydration product is charged to the column at a point which is at least ten plates above its bottom.

16. A process as claimed in any of the claims 1 and 3 to 15 wherein the washing liquid is fed between the 3rd plate below the withdrawal plate for pure dimethyl ether and the 3rd plate from the bottom of the distillation column.

17. A process as claimed in any of the claims 1 and 3 to 16 wherein the washing liquid is fed, in accordance with claim 1 (d), below the lowest feed plate for the dehydration product.

18. A process as claimed in any of the claims 1 and 3 to 17 wherein the dehydration product stream from the dimethyl ether synthesis reactor is united with the washing liquid stream before being fed to the dimethyl ether column.

19. A process as claimed in any of the claims 1 and 3 to 18 wherein the dehydration product from the dimethyl ether synthesis reactor and the washing liquid are charged separately to the same feed plate(s).

20. A process as claimed in any of the claims 1 and 3 to 19 wherein the methanol used as a washing liquid is at least in part crude methanol from a methanol synthesis.

**Revendications**

1. Procédé perfectionné de production d'éther diméthylique pur par déshydratation catalytique de méthanol à une température de 140-500°C et à une pression de 1 à 50 bars et par distillation du produit de déshydratation, caractérisé en ce que

a) le produit de déshydratation est introduit dans la colonne en vue de l'obtention d'éther diméthylique pur au-dessous du 25ème plateau (compté depuis la tête de la colonne) au niveau d'un ou plusieurs plateaux et au moins un plateau au-dessus de la base de la colonne,

b) l'éther diméthylique pur est déchargé à partir du 15ème plateau, de préférence du 10ème plateau (compté depuis la tête de la colonne) au niveau d'un ou plusieurs plateaux,

c) en ce qu'un distillat contenant des impuretés qui bouillent plus haut que l'éther de diméthyle et plus bas que le méthanol est déchargé au niveau d'un ou plusieurs plateaux se trouvant au moins 3 plateaux au-dessus de l'entrée (la plus haute) du mélange chargé contenant l'éther de diméthyle et au moins un plateau au-dessous du 15ème plateau (compté depuis la tête de la colonne), la distance entre le plateau de décharge de l'éther diméthylique pur et le plateau de décharge des impuretés étant de préférence au moins égale à 5 plateaux,

d) en plus du produit de déshydratation venant du réacteur de synthèse de l'éther de diméthyle, on utilise un liquide de lavage,

de telle sorte que pour une charge totale, y compris l'addition de liquide de lavage selon d) qui contient plus de 0 à 5 % en poids d'éther de diméthyle, on opère à un rapport de reflux de 1:0,2 à 1:100, de préférence de 1:1 à 1:25, pour une charge totale qui contient 20 à 80 % en poids d'éther de diméthyle, on opère à un rapport de reflux de 1:0,2 à 1:50, de préférence de 1:0,5 à 1:10 et notamment de 1:1 à 1:5, pour une charge totale qui contient plus de 5 et moins de 20 % en poids d'éther de diméthyle, on opère à un rapport de reflux de 1:0,2 à 1:50, de préférence de 1:0,4 à 1:25 et pour une charge totale qui contient plus de 80 % en poids et moins de 99 % en poids d'éther de diméthyle, on opère à un rapport de reflux de 1:0,2 à 1:50, de préférence de 1:0,2 à 1:10 et notamment de 1:0,4 à 1:5.

2. Procédé de production d'éther diméthylique pur par déshydratation catalytique de méthanol à une température de 140 à 500°C et à une pression à 1 à 50 bars et par distillation du produit de déshydratation, dans lequel le produit de déshydratation est introduit dans la colonne d'obtention de l'éther diméthylique pur au-dessous du 25ème plateau (compté depuis la tête de la colonne) au niveau d'un ou plusieurs plateaux et le produit de déshydratation est amené au moins un plateau au-dessus de la base de la colonne, l'éther diméthylique pur est déchargé à partir du 15ème plateau, de préférence à partir du 10ème plateau (compté depuis la tête de la colonne) au niveau d'un ou plusieurs plateaux, un distillat contenant des impuretés qui bouillent plus haut que l'éther de diméthyle et plus bas que le méthanol est déchargé au niveau d'un ou plusieurs plateaux se trouvant au moins 3 plateaux au-dessus de l'arrivée (la plus haute) du mélange chargé contenant l'éther de diméthyle et au moins un plateau au-dessous du 15ème plateau (compté depuis la tête de la colonne),

caractérisé en ce que pour une charge qui contient plus de 0 à 5 % en poids d'éther de diméthyle, on opère à un rapport de reflux de 1:0,2 à 1:<1, pour une charge qui contient 20 à 80 % en poids d'éther de diméthyle, on opère à un rapport de reflux de 1:0,2 à 1:<0,4 ou de 1:>5 à 1:50 et pour une charge qui contient plus de 5 à moins de 20 % en poids d'éther de diméthyle, on opère à un rapport de reflux de 1:0,2 à 1:<0,4 ou de 1:>25 à 1:50, ou bien pour une charge de plus de 80 % en poids à 99 % en poids d'éther de diméthyle, à un rapport de 1:>5 à 1:50.

3. Procédé suivant la revendication 1, caractérisé en ce que le liquide de lavage est le méthanol ou un mélange de méthanol et d'eau.

4. Procédé suivant la revendication 1, caractérisé en ce que le liquide de lavage est l'eau.

5. Procédé suivant les revendications 1 à 4, caractérisé en ce qu'on effectue la déshydratation avec un catalyseur du type $\gamma$-Al$_2$O$_3$ qui contient plus de 0 à 1 % en poids de SiO$_2$.

6. Procédé suivant les revendications 1 à 5, caractérisé en ce qu'on effectue la déshydratation avec un catalyseur du type $\gamma$-Al$_2$O$_3$ qui contient 0,001 à 0,5 % en poids de SiO$_2$.

7. Procédé suivant les revendications 1 à 6, caractérisé en ce qu'on effectue la déshydratation avec un catalyseur du type $\gamma$-Al$_2$O$_3$ qui contient 0,001 à 0,2 % en poids de SiO$_2$.

8. Procédé suivant les revendications 1 à 7, caractérisé en ce qu'on effectue la déshydratation à une température de 140 à 500 °C, de préférence à 140-450 °C et à une pression de 1 à 50 bars, de préférence de 3 à 25 bars.

9. Procédé suivant les revendications 1 à 8, caractérisé en ce qu'on effectue la déshydratation avec une charge du catalyseur (VSHL) de 0,2-16 1/1 h.

10. Procédé suivant les revendications 1 à 9, caractérisé en ce qu'on effectue la déshydratation avec une charge du catalyseur (VSHL) de 0,5 à 13,5 1/1 h.

11. Procédé suivant les revendications 1 à 10, caractérisé en ce que les composants légers déchargés à la tête de la colonne de distillation pour l'obtention d'éther diméthylique pur, qui contiennent généralement du CO$_2$, du N$_2$, des hydrocarbures légers et aussi de l'éther de diméthyle, sont lavés à contre-courant ou en courants parallèles, de préférence avec du méthanol.

12. Procédé suivant les revendications 1 à 11, caractérisé en ce que les impuretés déchargées au-dessous du point de décharge de l'éther diméthylique pur sont extraites dans un stripper latéral et en ce que l'éther diméthylique ainsi séparé est recyclé dans la colonne de distillation.

13. Procédé suivant les revendications 1 à 12, caractérisé en ce que le produit de déshydratation est introduit dans la colonne au-dessous du 25ème plateau au niveau d'un ou plusieurs plateaux, selon le nombre de plateaux entre le 50ème et le 25ème.

14. Procédé suivant les revendications 1 à 13, caractérisé en ce que le produit de déshydratation est amené au moins 5 plateaux au-dessus de la base de la colonne.

15. Procédé suivant les revendications 1 à 11, caractérisé en ce que le produit de déshydratation est amené au moins 10 plateaux au-dessus du fond de la colonne.

16. Procédé suivant les revendications 1 et 3 à 15, caractérisé en ce que le liquide de lavage est introduit dans la colonne de distillation entre le 3ème plateau inclusivement au-dessous du plateau de décharge de l'éther diméthylique pur et le 3ème plateau compté depuis le bas.

17. Procédé suivant les revendications 1 et 3 à 16, caractérisé en ce que le liquide de lavage suivant la revendication 1 (d) est amené au-dessous du plateau d'introduction le plus bas pour le produit de déshydratation.

18. Procédé suivant les revendications 1 et 3 à 17, caractérisé en ce que le produit de déshydratation venant du réacteur de synthèse de l'éther de diméthyle et le liquide de lavage sont réunis avant leur entrée dans la colonne d'éther de diméthyle.

19. Procédé suivant les revendications 1 et 3 à 18, caractérisé en ce que le produit de déshydratation venant du réacteur de synthèse de l'éther de diméthyle et le liquide de lavage sont chargés séparément mais sur le ou les mêmes plateaux d'injection.

20. Procédé suivant les revendications 1 et 3 à 19, caractérisé en ce que le méthanol utilisé comme liquide de lavage est au moins en partie du méthanol brut provenant d'une synthèse de méthanol.

Figur 1

_Figur 2_

_Figur 3_